# EUROPÄISCHE PATENTANMELDUNG

(11) **EP 2 568 277 A1**
(43) Veröffentlichungstag der Anmeldung: **13.03.2013**
(21) Anmeldenummer: 12183781.9
(22) Anmeldetag: 10.09.2012
(51) Int. Cl.: G01N 21/85

(54) **Verfahren zur Bestimmung einer Zusammensetzung einer plastifizierten Elastomermischung auf der Basis wenigstens eines polaren oder unpolaren Kautschuks**

(30) Priorität: 09.09.2011 DE 102011053478
(71) Anmelder: PHILIPPS-UNIVERSITÄT MARBURG, 35037 Marburg (DE); ContiTech AG, 30165 Hannover (DE)
(72) Erfinder: Koch, Martin, 35274 Kirchhain (DE); Lippert, Sina, 35037 Marburg (DE); Peters, Björn Ole, 35037 Marburg (DE); Sostmann, Stefan, 30855 Langenhagen (DE); Wichmann, Matthias, 35039 Marburg (DE); Wietzke, Steffen, 31275 Lehrte Hämelerwald (DE)
(74) Vertreter: Stumpf, Peter

(57) **Zusammenfassung**

Die Erfindung betrifft ein Verfahren zur Bestimmung einer Zusammensetzung einer plastifizierten Elastomermischung auf der Basis wenigstens eines polaren oder unpolaren Kautschuks, umfassend die folgenden Schritte:
- Bestrahlen der plastifizierten Elastomermischung auf der Basis des wenigstens einen polaren oder unpolaren Kautschuks in einem Prüfraum (3) eines THz-Spektrometers (2) mit elektromagnetischen Wellen im THz-Frequenzbereich;
- Bestimmen von wenigstens einer Ist-Korrelation zwischen der Frequenz der emittierten elektromagnetischen Wellen und einer dielektrischen Materialeigenschaft der Elastomermischung anhand von empfangenen elektromagnetischen Wellen;
- Vergleichen der Ist-Korrelationen mit Soll-Korrelationen mittels einer Auswerteeinheit, wobei in der Auswerteeinheit wenigstens eine Soll-Korrelation von wenigstens einer Elastomermischung hinterlegt ist;
- Ausgabe eines Informationssignals, wenn wenigstens eine Ist-Korrelation von der Soll-Korrelation der Elastomermischung abweicht.

## Beschreibung

Die Erfindung betrifft ein Verfahren zur Bestimmung einer Zusammensetzung einer plastifizierten Elastomermischung auf der Basis wenigstens eines polaren oder unpolaren Kautschuks gemäß Anspruch 1.

Zur Qualitätskontrolle stehen im Stand der Technik zahlreiche Verfahren und Vorrichtungen zur Verfügung, um eine Bestimmung einer Zusammensetzung eines Materials vorzunehmen. Trotz eines hohen Technologie- und Automatisierungsstandards werden etablierte Messverfahren bei der Produktion von Bauteilen aus fließfähigem Ausgangsmaterial, z. B. aus plastifiziertem Kunststoff, nachgeschaltet im Labor vorgenommen. Dies ist insbesondere auch bei der Fertigung von Produkten aus Elastomermischungen der Fall. Zu deren Überprüfung werden mikroskopische, chemisch-analytische und mechanische Verfahren eingesetzt. Diese sind zur Reduktion von Fertigungsausschuss nur bedingt geeignet, da hiermit erzielbare Messergebnis nicht zeitnah vorliegen. Chemisch-analytische Verfahren direkt in Fertigungsvorrichtungen zu integrieren, stellte sich bislang als ungeeignet heraus.

Weiterhin sind aus dem Stand der Technik Verfahren zur Laborbestimmung von Materialzusammensetzungen bekannt, die eine Terahertz-Spektroskopie einsetzen. So wird beispielsweise im Aufsatz "Analyzing sub-100-µm samples with transmission terahertz time domain spectroscopy" in der Fachzeitschrift "Optics Communications 282 (2009), Seiten 1304-1306 von M. Scheller et al. eine Bestimmung von einem Absorptionskoeffizienten und eines Brechungsindex durch eine transmissive Spektrometermessung beschrieben. Hierbei wurden Probekörper aus Silizium (HR Si) mit einer Dicke von 54 µm, Polyamidnylon 6 (PA6) mit einer Dicke von 25 µm und Quarzsubstrat mit einer Dicke von 700 µm geprüft, insbesondere mit breitbandiger Terahertz-Strahlung [THz-Strahlung]. Schon die Fertigung derart dünner und genau zu fertigender Probekörper ist für die praktische Anwendung zur Qualitätssicherung ungeeignet.

Ein anderer Aufsatz beschreibt unter der Überschrift "Determination of additive content in polymeric compounds with terahertz-domain spectroscopy" in Polymer Testing 26 (2007), Seiten 614-618, S. Wietzke et al. eine Laborbestimmung des Additivgehaltes in thermoplastischen Kunststoffen. Hierfür wurden Probekörper mit einer Dicke von 2 mm und glatter Oberfläche mittels THz-Wellen bestrahlt und mit der empfangen Strahlung ein Absorptionskoeffizient und ein Brechungsindex jeweils über einen Frequenzbereich bestimmt. Diese thermoplastischen Probekörper sind meist transparent für THz-Wellen. Die meisten Additive/Zuschlagstoffe weisen hingegen einen starken dielektrischen Kontrast auf. Bei einem größeren Volumenanteil im Kunststoff hebt sich die Mischung bei einer Spektroskopiemessung deutlich von der der geringer oder ungefüllten thermoplastischen Matrix ab. Aus Bauteilen mit hinreichender Baugröße lassen sich die hierfür notwendigen Probekörper zwar gewinnen, doch ist so nur eine nachgelagerte Laboruntersuchung möglich.

Ein weiterer Nachteil der nachgeschalteten Laborversuche besteht darin, dass eine Fertigungsvorrichtung bei einem Materialwechsel von einer Elastomermischung auf eine andere Elastomermischung pauschal mit der neuen Elastomermischung leergedrückt/gespült wird. Um sicherzustellen, dass die gesamte alte Elastomermischung aus der Fertigungsvorrichtung entfernt wurde, wird in der Regel eine größere Menge der neuen Elastomermischung aufgewandt als notwendig. Hierdurch entstehen Materialkosten, Aufwand, Entsorgungskosten und die Umwelt wird belastet.

Aufgabe der Erfindung ist es deshalb, die Nachteile des Standes der Technik zu beseitigen, und ein Verfahren zu entwickeln, das in den Fertigungsprozess integrierbar ist und zuverlässige zeitnahe Ergebnisse hinsichtlich der Zusammensetzung einer Elastomermischung liefert. Die Fix- und Betriebskosten von diesem sollen gering sein, eine Schädigung der Elastomermischung soll nicht auftreten und es soll ein hoher Automatisierungsgrad erreicht werden.

Hauptmerkmale der Erfindung sind im kennzeichnenden Teil von Anspruch 1 angegeben. Ausgestaltungen sind Gegenstand der Ansprüche 2 bis 11.

Die Erfindung betrifft ein Verfahren zur Bestimmung einer Zusammensetzung einer plastifizierten Elastomermischung auf der Basis wenigstens eines polaren oder unpolaren Kautschuks, umfassend die folgenden Schritte:
- Bestrahlen der plastifizierten Elastomermischung auf der Basis des wenigstens einen polaren oder unpolaren Kautschuks in einem Prüfraum eines THz-Spektrometers mit elektromagnetischen Wellen im THz-Frequenzbereich;
- Bestimmen von wenigstens einer Ist-Korrelation zwischen der Frequenz der emittierten elektromagnetischen Wellen und einer dielektrischen Materialeigenschaft der Elastomermischung anhand von empfangenen elektromagnetischen Wellen;
- Vergleichen der Ist-Korrelationen mit Soll-Korrelationen mittels einer Auswerteeinheit, wobei in der Auswerteeinheit wenigstens eine Soll-Korrelation von wenigstens einer Elastomermischung hinterlegt ist;
- Ausgabe eines Informationssignals, wenn wenigstens eine Ist-Korrelation von der Soll-Korrelation der Elastomermischung abweicht.

Die zu untersuchende Elastomermischung kann unter anderem durch Vorgänge des Weichmachens von Kunststoffen unter Anwendung von Druck, Kneten, Extrudieren, Mastikation, Wärme und/oder Weichmachern (Plastifizier-, Plastifizierungs-, Plastifikationsmittel, Plastifikatoren) plastifiziert werden (siehe RÖMPP Chemielexikon Online, Version 3.16).

Der Prüfraum für Spektroskopiemessungen ergibt sich zumeist allein dadurch, dass elektromagnetische Wellen richtungsgebunden emittiert und ortsgebunden empfangen werden. Die elektromagnetischen Wellen können vom Spektrometer, bzw. von einem Emitter emittiert werden. Das Spektrometer könnte hierfür einen oder mehrere Emitter aufweisen. Weiterhin kann die elektromagnetische Strahlung mit einem oder mehreren Empfängern empfangen werden. Unterschiedliche Anordnungen sind hierbei möglich. So können die Empfänger und Emitter an der gleichen Position angeordnet sein, wodurch eine reflektive Messung erfolgen kann. Letztere ist auch durch die Nutzung eines Strahlteilers oder mittels einer Anordnung von Emitter und Empfänger in einem Winkel zueinander durchführbar. Möglich wäre auch eine abgeschwächte Totalreflexion (engl.: attenuated total reflection spectroscopy, ATR). Bei diesem Verfahren wird über ein angekoppeltes Prisma die Wechselwirkung des evaneszenten Feldes der elektromagnetischen Welle mit dem Material gemessen. Berücksichtigt wird hierbei allerdings nur eine geringe Eindringtiefe im Mikrometerbereich.

Besonders zu bevorzugen ist jedoch eine gegenüberliegende Anordnung von Emitter und Empfänger auf zwei unterschiedlichen Seiten des Prüfraums, insbesondere auf zwei unterschiedlichen Seiten einer Schlitzdüse und/oder eines Bypasses. Dies ist einfacher händelbar als eine Reflexionsanordnung. Der Abstand zwischen Empfänger und Emitter kann dann so groß gewählt werden, dass die Elastomermischung problemlos durch den Durchgang strömen kann.

Der von den elektromagnetischen Wellen zwischen dem Emitter und dem Empfänger durchdrungene Raum kann dann den Prüfraum bilden. Mithin kann der Prüfraum als ein Raum, in welchem Material mittels einer Spektroskopiemessung untersuchbar ist, beschrieben werden. Die Größe des Prüfraums ist im Wesentlichen abhängig von der Messart, z. B. transmissiv und/oder reflektiv, und der Anordnung von Emitter und Empfänger.

Geeignet zur Bestrahlung der im Prüfraum befindlichen Elastomermischung ist vor allem eine gepulste Emittierung von elektromagnetischen Wellen. Dies erlaubt eine Messung einer zeitlichen Verzögerung der elektromagnetischen Wellen, hervorgerufen durch die Elastomermischung, im Vergleich zu einem Referenzsignal. Diese Verzögerung ist abhängig von der Ausbreitungsgeschwindigkeit der elektromagnetischen Wellen und somit von den dielektrischen Materialeigenschaften der Elastomermischung im Prüfraum. Außerdem kann durch die Verwendung von THz-Pulsen ein breitbandiges Spektrum im Single-Shot-Modus erhalten werden. Eine schnelle und genaue Bestimmung der Rezepturzusammensetzung ist erreichbar, wenn die Bestrahlung der durch den Prüfraum strömenden Elastomermischung mit breitbandigen elektromagnetischen Wellen erfolgt. Dies erlaubt eine Bestimmung von einer Vielzahl von Wertepaaren jeweils bestehend aus einer Frequenz und einer dielektrischen Materialeigenschaft mit nur einem einzigen Impuls emittierter elektromagnetischer Wellen.

Die THz-Strahlung eignet sich besonders gut, um auch die relativ stark absorbierenden Elastomere zu untersuchen, da deren Eindringtiefe in das Material noch ausreichend groß ist. Außerdem sprechen bei der THz-Strahlung noch genügend Polarisationsmechanismen der Dielektrika an. Hierdurch reagieren die Absorption und der Berechungsindex bei Dosierungsfehlern von Zuschlagsstoffen besonders deutlich. Dies macht Dosierungsfehler sehr gut erkennbar. Gerade auch bei Elastomermischungen auf Kautschukbasis ist so eine wirksame Qualitätskontrolle erzielbar. Dies konnte in umfangreichen Messreihen an Elastomermischungen auf Basis von Ethylen-Propylen-Dien-Kautschuk (EPDM), Naturkautschuksystemen (NR), Acrylnitril-Butadien-Kautschuk (NBR) und Chlorbutadien-Kautschuk (CR) nachgewiesen werden.

Zur Bestimmung der Zusammensetzung der Elastomermischungen hat sich in Untersuchungen ein Frequenzspektrum der emittierten elektromagnetischen Wellen von 0,2-0,8 THz als besonders geeignet erwiesen. THz-Wellen vereinen eine hohe Eindringtiefe mit einer submillimeter genauen räumlichen Auflösung. Zudem reagieren die Messergebnisse nur wenig auf die im Prüfraum üblicherweise vorliegenden Temperatur- und Druckveränderungen. Hierdurch ist es möglich, die Messeinrichtung in unklimatisierten Bereichen und damit direkt in der Produktion einzusetzen.

Vor allem die THz-Zeitbereichsspektroskopie, auch Terahertz Time Domain Spectroscopy [THz-TDS] genannt, ist hervorragend einsetzbar. Deren breitbandige THz-Strahlung kann hierbei z. B. mit ultrakurzen Laserimpulsen erzeugt und kohärent detektiert werden. Amplitude und Phase der THz-Strahlung können dabei gleichzeitig unabhängig voneinander gemessen werden. Vorteilhaft ist weiterhin, dass die Messung bei Raumlicht und ohne Kühlung des Detektors durchführbar ist. Zusätzlich wirkt die THz-Strahlung aufgrund ihrer geringen Photonenenergie nicht ionisierend. Untersuchungen im Auftrag des Bundesamtes für Strahlenschutz unter Federführung der Physikalisch Technischen Bundesanstalt haben darüber hinaus zu dem Ergebnis geführt, dass die Gefährdung durch THz-Strahlung um einen Faktor 1000 - 10000 unter den deutschen Grenzwerten liegt (Studie "Gentoxische Effekte von THz-Strahlung in vitro"). Die Lichtwege der die THz-Strahlung erzeugenden Laserimpulse können entweder gekapselt oder fasergeführt sein und sind dann für den Benutzer ebenfalls ungefährlich.

Eine Soll-Korrelation und/oder eine Ist-Korrelation könnten aus einem einzelnen Wertepaar bestehen, das eine dielektrische Materialeigenschaft bei einer bestimmten Frequenz angibt. Dementsprechend müsste nur eine elektromagnetische Wellenlänge im THz-Frequenzbereich emittiert werden. Hierfür notwendige Emitter sind preiswert. Zum Beispiel könnte eine so genannte Dauerstrichmessung (engl. Contious wave [cw]) mit wenigstens einer Laserdiode, die im THz-Frequenzbereich emittiert, durchgeführt werden. Diese lässt sich z.B. durch das Mischen von 2 oder mehreren Laserdioden im optischen Bereich, durch elektronische Mischprozesse oder mit Quanten-Kaskaden-Lasern mit frei einstellbaren Intra-Band-Übergängen realisieren. Vorzugsweise bestehen die Korrelationen jedoch aus einer Vielzahl an Wertepaaren, sodass einer Vielzahl an bestimmten Frequenzen jeweils ein Wert einer dielektrischen Materialeigenschaft zugeordnet ist. Besonders bevorzugt beschreibt die Soll-Korrelation und/oder eine Ist-Korrelation einen lückenlosen Verlauf einer dielektrischen Materialeigenschaft über ein Frequenzspektrum. Ein solcher lückenloser Verlauf ist beispielsweise durch Approximation, Interpolation, Ausgleichsrechung oder andere Näherungsverfahren aus mehreren diskreten Wertepaaren erstellbar.

Hinterlegt werden könnte die Soll-Korrelation in der Auswerteeinheit schließlich als Tabelle, Graph oder Funktion. Die Soll-Korrelation und oder die Ist-Korrelationen beschreiben somit dielektrische Materialeigenschaften einer Elastomermischung auf Kautschukbasis. Zu den polaren und unpolaren Kautschuken zählen insbesondere:

| **unpolare Kautschuke** | | **polare Kautschuke** | |
|---|---|---|---|
| NR | Naturkautschuk, natürliches | NBR | Nitrilkautschuk |
| | Polyisopren | | |
| IR | synthetisches Polyisopren | HNR | Halonitrilkautschuk |
| SBR | Styrolbutadienkautschuk | CR | Chloroprenkautschuk |
| S-SBR | lösungspolymerisierter | CM | Chloriertes Polyethylen |
| | Styrolbutadienkautschuk | | |
| E-SBR | emulsionspolymerisierter | CSM | Chlorsulfoniertes Polyethylen |
| | Styrolbutadienkautschuk | | |
| BR | Butandienkautschuk | ACM | Acrylat-Kautschuk |
| HBR | Halobutylkautschuk | EVA | Ethylen-Vinylacetat-Copolymere |
| PNR | Polynorbornen | FPM | Fluorkautschuke |
| IIR | Isopren-Isobutylen-Copolymere | MQ | Methylsilicon-Kautschuke |
| BIIR | Brombutylkautschuk | MVQ | Vinylsilicon-Kautschuke |
| CIIR | Chlorbutylkautschuk | MFQ | Polyfluorsilicone |
| EPM | Ethylen-Propylen-Kautschuk | MPVQ | Phenyl-Vinyl-Silicon-Kautschuk |
| EPDM | Ethylen-Propylen-Dien-Kautschuk | AU, EU | Polyester-Polyisocyanat-Prepolymere |
| | | CO, | ECO Epichlorhydrinkautschuk |
| | | TM | Polysulfidkautschuke |

Diese tabellarisch aufgeführten Kautschuke können mit unterschiedlichen Zusatzstoffen zu Elastomermischungen vermischt werden.

Zusatzstoffe erfüllen unterschiedliche Aufgaben. So kommen Füllstoffe wie Kieselsäure, Ruß, Silikate, Oxide, Carbonate und dergleichen. zum Einsatz. Hinzu kommen Weichmacher, Ozonschutzmittel, Alterungsschutzmittel, Mastikationshilfsmittel und weitere Aktivatoren. Außerdem werden Vernetzungssysteme bestehend aus Vernetzer, Schwefel, Peroxid, Beschleuniger und Verzögerer in die Elastomermischung eingebracht.

Gerade bei der Mischung von Elastomermischungen können Fehler auftreten, die mit erfindungsgemäßem Verfahren detektierbar sind. Insbesondere aufgrund der großen Durchsätze und hohen Produktionsstückzahlen pro Zeit bei einer Fertigung von Elastomerteilen kann durch die prozessbegleitende Überwachung der Zusammensetzung der Elastomermischung sehr viel Ausschuss verhindert werden. Mitunter vermeidet dies gar Rückrufaktionen von ausgelieferten Teilen. Zusätzlich ist eine Prozessdokumentation im Rahmen des Qualitätsmanagements möglich.

Der Vergleich zwischen Ist-Korrelation und Soll-Korrelation kann anhand eines Vergleichs zwischen den Werten der dielektrischen Materialeigenschaft der Ist-Korrelation und der Soll-Korrelation an wenigstens einer Frequenz erfolgen. Bevorzugt erfolgt er jedoch an mehreren Frequenzwerten und besonders bevorzugt lückenlos über ein Frequenzspektrum. Eine Abweichung sollte dabei nur detektiert werden, wenn diese größer als die Messtoleranz der Messeinrichtung oder als eine festgelegte Materialspezifikation ist.

Um einen Fehler in der Rezepturzusammensetzung sicher detektieren zu können, könnte jedoch auch vorgesehen sein, dass in der Auswerteeinheit Toleranzgrenzen für maximale Abweichungen von den Soll-Korrelationen hinterlegt sind, und das Verfahren folgenden Schritt umfassen:
- Ausgabe eines Informationssignals wenn wenigstens eine Ist-Korrelation um mehr als die Toleranzgrenze von der Soll-Korrelation abweicht.

Mithin kann durch das Verfahren eine Messung zur Bestimmung einer dielektrischen Materialeigenschaft über einen Frequenzbereich von elektromagnetischen Wellen vorgenommen werden. Durch einen Vergleich dieses Messergebnisses (Ist-Korrelation) mit der Soll-Korrelation kann festgestellt werden, ob das Messergebnis von der Soll-Korrelation abweicht. Ist eine Abweichung vorhanden, kann diese vor allem zur Detektion einer falschen Zusammensetzung der Elastomermischung genutzt werden.

Entsprechend ermöglicht das Verfahren eine praxisnahe genaue Beurteilung der korrekten Zusammensetzung der Elastomermischung. Zudem erlaubt es eine kontinuierliche Messung und es liegen keine Messlücken zwischen einzelnen Stichproben vor. Somit kann frühzeitig festgestellt werden, ob die Qualität einer Elastomermischung stimmt, und die Produktion von Ausschuss lässt sich reduzieren. Eine personal- und zeitintensive Probenpräparation und Laboruntersuchung ist nicht notwendig.

Das verfahrensgemäß ausgegebene Informationssignal, kann jeweils verschiedene Informationen beinhalten. Sofern nur eine Abweichung von der Soll-Korrelation bestimmt wurde, ist das Informationssignal in der Regel ein Fehlersignal. Dieses kann eine reine Visualisierung für Personen sein, oder aber es wird von einer Steuereinrichtung weiterverwertet, zum Beispiel zum Durchführen eines Produktionsstopps.

Außerdem kann ein schneller Rezepturwechsel mit geringen Kosten und guter Ökobilanz durchgeführt werden, indem der Zeitpunkt des Vorliegens einer neuen Elastomermischung entsprechend der Spezifikation im Prüfraum genau bestimmt werden kann. Mit dem Verfahren erzielte Daten können zusätzlich an moderne Prozessregelsysteme übertragen werden, um so Qualitätsschwankungen bei der Fertigung zu vermeiden und Einsparungen hinsichtlich des Rohmaterial- und des Energieverbrauchs zu erzielen. All dies ist mit einem geringen Personalaufwand erreichbar. Eine effiziente Qualitätskontrolle mit engen Qualitätstoleranzen und gut zertifizierbaren Bauteilen aufgrund lückenloser Überwachung wird hiermit realisierbar.

Eine für das Verfahren notwendige Messeinrichtung kann bedarfsgerecht für Ihre Aufgaben ausgelegt sein und deren hoher Automatisierungsgrad hält die Personalkosten gering. Die Fixkosten sind so überschaubar und die laufenden Kosten niedrig. Für einen einfachen Einsatzort ist es gegebenenfalls sogar ausreichend, ein QuasiTime-Domain Spektrometer einzusetzen, welches THz-Pulse mit Multimodelaserdioden erzeugt [Aufsatz "Terahertz quasi time domain spectroscopy" in der Fachzeitschrift "Optics Express 17 (2009), Seiten 17723-17733 von M. Scheller und Martin Koch]. Die Fixkosten wären dementsprechend besonders gering. Damit sind Multimodelaserdioden langfristig als preiswerte THz-Emitter einsetzbar.

Eine wichtige Ausgestaltung der Erfindung sieht vor, dass eine erste Ist-Korrelation und eine zugehörige erste Soll-Korrelation jeweils eine spektrale Dispersion einer dielektrischen Materialeigenschaft beschreiben.

Demgemäß sollte dann im Verfahren eine erste Ist-Korrelation zwischen der Frequenz der emittierten elektromagnetischen Wellen und einer spektralen Dispersion einer dielektrischen Materialeigenschaft der Elastomermischung bestimmt werden. Hierdurch ist erkennbar, wie stark die dielektrische Materialeigenschaft von der Frequenz der elektromagnetischen Wellen abhängig ist. Die Analyse des dispersiven Verlaufs, insbesondere die der Steigung des Brechungsindex, erlaubt eine Detektion einer Rezepturabweichung. Zusätzlich kann eine Eingrenzung auf mögliche falsch dosierte Rezepturbestandteile oder sogar direkt eine Identifizierung des falsch dosierten Rezepturbestandteils erfolgen. Besonders stark äußert sich beispielsweise die Änderung einer Ausbreitungsgeschwindigkeit von elektromagnetischen Wellen in Elastomermischungen bei einer Dosierungsabweichung von stark polarisierenden Bestandteilen. Zu diesen stark polarisierenden Bestandteilen zählen zum Beispiel die häufig eingesetzten Zuschlagstoffe Ruß und Schwefel.

Dabei kann die spektrale Dispersion eine frequenzabhängige Änderung einer Ausbreitungsgeschwindigkeit über der Frequenz, von sich in der Elastomermischung ausbreitenden elektromagnetischen Wellen umfassen. Weicht die spektrale Dispersion der Ausbreitungsgeschwindigkeit stark von der ersten Soll-Korrelation ab, kann beispielsweise nicht nur eine Ja/Nein-Aussage hinsichtlich einer falschen Zusammensetzung der Elastomermischung gemacht werden, sondern auch darauf zurückgeschlossen werden, dass mit hoher Wahrscheinlichkeit ein Rezepturbestandteil falsch dosiert ist. In Elastomermischungen könnte dies beispielsweise auf den Zuschlagstoff Ruß zutreffen. Angegeben wird die Ausbreitungsgeschwindigkeit typischerweise mit einem Brechungsindex.

Besonders bevorzugt umfasst die spektrale Dispersion einen Differenzenquotienten aus einem Brechungsindex der Elastomermischung und der Frequenz von sich in der Elastomermischung ausbreitenden elektromagnetischen Wellen. Der Brechungsindex ist die typische physikalische Größe zur Beschreibung einer Ausbreitungsgeschwindigkeit von elektromagnetischen Wellen in Materialien. Er gibt das Verhältnis der Ausbreitungsgeschwindigkeit einer elektromagnetischen Welle im Vakuum zu der Ausbreitungsgeschwindigkeit im Material an. Der Brechungsindex eignet sich deshalb nicht nur für eine Vergleichsmessung mit Hilfe von Korrelationen, sondern er kann auch mit absoluten Werten verglichen werden, die beispielsweise in einer Datenbank hinterlegbar sind. Die Datenbank kann dabei Teil der Auswerteeinrichtung sein. Zudem eignet sich der Brechungsindex gerade im THz-Frequenzbereich sehr gut als dielektrischer Parameter zur Kontrolle der Zusammensetzung von Elastomermischungen auf Kautschukbasis.

Der Differenzenquotient kann über einen größeren Frequenzbereich ausgebildet werden, zum Beispiel zwischen zwei Messpunkten. Er könnte jedoch auch über einen infinitesimal kleinen Frequenzabschnitt gebildet werden, sodass er näherungsweise einem Differentialquotienten in einem Punkt entspricht. Hierfür könnte auch eine direkte Ableitung einer Brechungsindexfunktion nach der Frequenz erfolgen. Ein über einen größeren Frequenzbereich ausgebildeter Differenzenquotient eignet sich in der Praxis jedoch meist besser, da dieser Schwankungen der Steigung des Brechungsindexes, z. B. aufgrund statistischer Fehler, innerhalb des Frequenzbereichs außer Betracht lässt. Außerdem ist der Rechenaufwand für die Berechnung gering.

Ein anderer wichtiger Parameter des Verfahrens kann eine zweite Ist-Korrelation und eine zugehörige zweite Soll-Korrelation sein, die jeweils eine Absorption elektromagnetischer Wellen durch die Elastomermischung in Abhängigkeit von der Frequenz beschreiben. Dementsprechend sollte das Verfahren eine zweite Ist-Korrelation zwischen der Frequenz der emittierten elektromagnetischen Wellen und einer Absorption der elektromagnetischen Wellen durch die Elastomermischung bestimmen.

Die Absorption ist eine dielektrische Materialeigenschaft, welche die Durchdringbarkeit des Materials durch elektromagnetische Wellen angibt. Auch bei einer Spektroskopiemessung und einem Vergleich einer hierdurch bestimmten Ist-Korrelation der Absorption mit einer Soll-Korrelation der Absorption kann festgestellt werden, dass die Zusammensetzung des Materials nicht stimmt. Physikalische Größe für die Absorption kann beispielsweise der Absorptionskoeffizient oder der Extinktionskoeffizient sein.

Je mehr dielektrische Materialeigenschaften betrachtet werden, desto eher sind Fehler in der Elastomermischung detektierbar. Dies ist insbesondere dann relevant, wenn wenigstens zwei Bestandteile der Elastomermischung falsch dosiert sind, die in Summe gegenläufige dielektrische Veränderungen bewirken. So ist es möglich, dass eine Elastomermischung trotz falscher Dosierung zweier Bestandteile die gleiche Absorption aufweist. Dafür unterscheidet sich dann mit hoher Wahrscheinlichkeit die Dispersion einer dielektrischen Materialeigenschaft der falschen Rezeptur von der Soll-Korrelation. Auch derartige falsche Zusammensetzungen des Materials können so aufgedeckt werden.

Gemäß einer weiteren Ausbildung des Verfahrens ist vorgesehen, dass eine dritte Ist-Korrelation und eine zugehörige dritte Soll-Korrelation eine Ausbreitungsgeschwindigkeit von elektromagnetischen Wellen in der Elastomermischung in Abhängigkeit von der Frequenz beschreiben. Bei der Bestimmung der Ist-Korrelation sollte dann eine Korrelation zwischen der Frequenz der emittierten elektromagnetischen Wellen und einer Ausbreitungsgeschwindigkeit der elektromagnetischen Wellen in der Elastomermischung bestimmt werden.

Die dielektrische Materialeigenschaft der Ausbreitungsgeschwindigkeit wird meist mit der physikalischen Größe des Brechungsindexes angegeben. Sie kann zum Beispiel durch eine Transmission der elektromagnetischen Wellen durch die Elastomermischung bestimmt werden, indem die von der Elastomermischung verursachte Zeitverzögerung erfasst wird. Die Ausbreitungsgeschwindigkeit eignet sich durch ihre Abhängigkeit von den dielektrischen Materialeigenschaften ebenfalls für die Detektion von Dosierungsfehlern der Bestandteile einer Elastomermischung. Dabei reagiert die Ausbreitungsgeschwindigkeit schon bei geringen Dosierungsabweichungen. In Kombination mit der ersten und/oder zweiten Soll-Korrelation ist so nahezu jede Rezepturabweichung detektierbar. Auch geringe Änderungen von Zuschlagstoffen in Elastomermischungen wie zum Beispiel Ruß, Schwefel, Zinkoxid, Beschleuniger, Fettsäuren und Stabilisatoren können so wirksam erkannt werden.

Sehr gut eignet sich das Verfahren auch dann, wenn die plastifizierte Elastomermischung während der Bestimmung der Ist-Korrelationen durch einen Durchgang des Prüfraums hindurch geleitet wird. Mithin kann das Verfahren unmittelbar in der Fertigung eingesetzt werden, indem eine plastifizierte Elastomermischung vor der Weiterverarbeitung durch den Durchgang geleitet wird. Hierbei kann die Elastomermischung direkt im Fertigungsprozess hinsichtlich der korrekten Elastomermischung beurteilt werden.

Für Elastomere hat der Durchgang bevorzugt eine Querschnittsbreite von weniger als 2,5 mm, und beträgt besonders bevorzugt 1 +- 0,2 mm. Die zu überbrückende Distanz der elektromagnetischen Wellen im Material bei einer transmissiven Messung mit dem THz-Spektrometer kann dann ebenfalls maximal 2,5 mm betragen. Gleichzeitig ist der Spalt groß genug, um von der plastifizierten und fließfähigen Elastomermischung durchströmt werden zu können. Die Querschnittslänge kann hingegen größer gewählt werden.

Besonders vorteilhaft ist es, wenn ein Extruder mit dem Durchgang des Prüfraums strömungsverbunden ist. Insbesondere kann der Extruder mit einer Eintrittsseite des Durchgangs des Prüfraums strömungsverbunden sein. Die plastifizierte Elastomermischung kann dann durch den Durchgang des Prüfraums von der Eintrittsseite zu einer Austrittsseite des Durchgangs geleitet werden.

Mittels eines Extruders werden Elastomermischungen plastifiziert, durchmischt und mit einem Druck beaufschlagt abgegeben. Derartige Extruder haben meist eine oder zwei Schneckenwellen, welche die Elastomermischung in einen rohrförmigen Körper fördert, an dessen Ende ein Materialausgang angeordnet ist. Deshalb wird dieser Extrudertyp auch als Ein- oder Doppelschneckenextruder bezeichnet. Durch eine Verbindung des Extruders mit dem Prüfraum sind Abweichungen von der Elastomermischung zu einem frühen Zeitpunkt eines Fertigungsprozesses detektierbar. Außerdem ist ab einem Prozessschritt hinter dem Extruder kaum noch eine Änderung der Elastomermischung herbeiführbar, da die Elastomermischung im direkten Anschluss an den Extruder in ein Objekt, Produkt oder ähnliches überführt wird.

Besonders vorteilhaft ist es, wenn der Durchgang des Prüfraums Teil eines Strömungskanals ist. Für eine Fertigung von Objekten aus einer Elastomermischung kann ein solcher Strömungskanal als Heißkanal ausgebildet sein. Der Strömungskanal kann sich unmittelbar an den Extruder anschließen und ist geeignet dazu, die Elastomermischung weiterzuleiten. Durch die Ausbildung des Durchgangs des Prüfraums kann die plastifizierte Elastomermischung vollständig oder teilweise durch den Prüfraum geleitet werden. Dabei muss der Prüfraum nicht an den Extruder angrenzen, sondern kann über den Strömungskanal indirekt mit dem Extruder strömungsverbunden sein.

Die durch den Durchgang des Prüfraums strömende Elastomermischung wird dem Strömungskanal vorzugsweise wieder zugeführt. Dadurch wird kein Material für die Durchführung der Messung verbraucht.

Möglich wäre jedoch auch eine Abzweigung einer kleinen Menge der Elastomermischung aus dem Strömungskanal für die Messung, die nicht zurück in den Strömungskanal geführt wird. Die Austrittsseite des Durchgangs des Prüfraums könnte dafür mit einer Sammeleinrichtung strömungsverbunden sein. Dies erlaubt auch eine Durchführung des Verfahrens, wenn für eine Installation einer Messeinrichtung platzbedingt keine Verbindung der Austrittsseite mit dem Strömungskanal möglich ist. Damit kein Abfall entsteht, kann die Austrittsseite des Durchgangs jedoch auch mit einem Materialspeicher des Extruders strömungsverbunden sein, um die Elastomermischung zurückzuführen.

Sehr gut geeignet zur Integration der Messeinrichtung in die Fertigungsvorrichtung ist es, wenn der Durchgang des Prüfraums als Teil einer Schlitzdüse und/oder als Teil eines Bypass ausgebildet ist. Die Schlitzdüse verjüngt den Strömungsquerschnitt auf eine Schlitzbreite. Dies vereinfacht die Messung mit THz-Strahlung erheblich, da letztere in vielen Elastomermischungen eine nur begrenzte Eindringtiefe erreicht. Die Schlitzdüse kann in einem Hauptströmungskanal hinter dem Extruder angeordnet sein. Entweder verjüngt sie diesen insgesamt auf die Schlitzbreite, oder aber der Schlitz sitzt nur innerhalb des Hauptströmungskanals, erstreckt sich jedoch nicht über dessen gesamten Querschnitt. Der Schlitz kann dabei auch teilweise offen ausgebildet sein. Dies verbessert dessen Durchströmbarkeit und Selbstreinigung. Die Schlitzdüse könnte jedoch gleichermaßen im Bypass angeordnet sein. Ein Bypass ist als separater paralleler Kanalabschnitt zum Hauptströmungskanal ausgebildet. Dies erlaubt unter anderem eine flexiblere Adaption sowie Positionierung der Messeinrichtung.

Somit kann durch die Schlitzdüse und/oder den Bypass der Querschnitt des Strömungskanals zwischen dem Extruder und dem Zielort hinreichend groß und nur in bedingtem Maße abhängig von der Messeinrichtung ausgelegt werden. Außerdem kann die Messeinrichtung vorkonfiguriert installiert werden und deren Aufbau, insbesondere die Positionen von Emittern und Empfängern, sind unabhängig vom Hauptströmungskanal. Es ist sogar möglich, eine solche Messeinrichtung nachträglich zu installieren.

Zur Kalibrierung kann im Rahmen des Verfahrens folgender Schritt vorgesehen werden:
- Hinterlegen einer ermittelten Ist-Korrelation als Soll-Korrelation einer Referenzmischung in der Auswerteeinheit.

Hierdurch ist es sehr einfach möglich, neue Elastomermischungen auf deren dielektrische Materialeigenschaften zu überprüfen und die Daten als Ist-Korrelation einer Referenzmischung für zukünftige Bestimmungen in der Auswerteeinheit zu hinterlegen. Unter anderem kann so eine Neueinrichtung einer Messeinrichtung zur Durchführung des Verfahrens erfolgen, bei der erstmals eine Soll-Korrelation in der Auswerteeinheit hinterlegt wird. Außerdem könnten Soll-Korrelationen mehrerer Elastomermischungen hinterlegt werden, um beispielsweise Objekte aus unterschiedlichen Elastomermischungen mit der gleichen Fertigungsvorrichtung herstellen und dabei die divergierenden Elastomermischungen überwachen zu können.

Das Verfahren eröffnet zusätzlich die Möglichkeit nach dem Vergleichen der Ist-Korrelationen mit den Soll-Korrelationen zwischen diesen ermittelte Abweichungen anhand von definierten Fehlereingrenzungskriterien zu interpretieren. Auf diese Weise kann eine Eingrenzung auf eine Gruppe von möglichen falsch dosierten Bestandteilen in der Elastomermischung und/oder Detektion eines falsch dosierten Bestandteils in der Elastomermischung mit Hilfe der Fehler-Eingrenzungskriterien erfolgen. Anschließen könnte sich dann eine Ausgabe eines Fehlersignals mit Angabe des oder der wahrscheinlich falsch dosierten Bestandteile.

Die Eingrenzungskriterien können Informationen über die in der Rezeptur enthaltenen Zuschlagstoffe sein. Ein solches Eingrenzungskriterium wäre beispielsweise die Polarisierbarkeit der Bestandteile/Zuschlagstoffe. Außerdem bestehen Zusammenhänge zwischen dem Absorptionskoeffizienten, dem absoluten Brechungsindexwert und der Steigung des Brechungsindexes. Nachstehende Tabelle zeigt gemessene Zusammenhänge von sechs wichtigen Zuschlagstoffen in Elastomermischungen auf NR-Basis mit den Zuschlagstoffen Ruß, Schwefel, Vernetzer (Zinkoxid [ZnO]), Vernetzungsbeschleuniger, Fettsäuren und Stabilisatoren, bei einer relativen Erhöhung von deren Anteil um 20%. Die Messwerte basieren auf einer Frequenz der elektromagnetischen Wellen von 1 THz:

| | Auswirkung bei einer relativen Rezepturanteilerhöhung um 20%, bei f = 1 THz | |
|---|---|---|
| Rezepturanteil | Änderung des Brechungsindexes KI3-KS3 = Δn | Änderung der Dispersion des Brechungsindexes = Δ(Δn/Δf) |
| Ruß | 0,12 | 26 fs |
| Schwefel | -0,04 | 15 fs |
| Zinkoxid [ZnO] | 0,03 | 3 fs |
| Beschleuniger | -0,025 | 8 fs |
| Fettsäuren | 0,01 | -3 fs |
| Stabilisatoren | -0,005 | -4 fs |

Ein Fehlereingrenzungskriterium könnten somit diese Zusammenhänge sein. Beispielsweise steigt der Absolutwert des Brechungsindexes (Δn) bei einer Erhöhung von Ruß merklich an und auch die Änderung der Dispersion Δ(Δn/Δf) nimmt hierbei sehr stark zu. Dahingegen sinkt der Brechungsindex (Δn) geringfügig und die Änderung der Dispersion Δ(Δn/Δf) steigt deutlich weniger bei einer Erhöhung des Schwefelanteils. Weiterhin steigt der Brechungsindex (Δn) bei einer Erhöhung des Zinkoxidanteils leicht und die Änderung der Dispersion Δ(Δn/Δf) nimmt nur geringfügig zu. Anhand des Betrags und der Vorzeichen der Abweichungen können somit Gruppen von Zuschlagstoffen als ursächlich für eine Abweichung der Ist-Korrelationen von den Soll-Korrelationen ausgeschlossen werden. Wäre tatsächlich der Rußanteil in dem Material erhöht und die Zuschlagstoffe gemäß obiger Tabelle enthalten, ergäbe sich eine Erhöhung des absoluten Brechungsindexes (Δn) bei 1 THz und eine starke Erhöhung der Steigung des Brechungsindex Δ(Δn/Δf). Damit könnte für die Abweichungen der Ist-Korrelationen von den Soll-Korrelationen wenigstens ausgeschlossen werden, dass hierfür die Anteile des Zinkoxids, der Beschleuniger, der Fettsäuren und der Stabilisatoren verantwortlich sind, denn bei diesen würden sich der Brechungsindex (Δn) und dessen Steigung Δ(Δn/Δf) nur geringfügig bzw. sogar gegenläufig ändern.

Weiterhin ändert sich der Brechungsindexverlauf (Δn) bei Variation des Rußanteils am deutlichsten. Dies betrifft nicht nur die Absolutwerte im Vergleich zweier Rezepturen, sondern auch die Änderung der Dispersion Δ(Δn/Δf). Mit steigendem Rußgehalt verläuft der Brechungsindex (Δn) dabei dispersiver. Als Maß hierfür dient idealer Weise ein Differenzenquotient (Δn/Δf) des Brechungsindexes über einen Frequenzbereich. Während sich der Differenzenquotient zwischen den Variationsstufen für Ruß und Schwefel nahezu verdoppelt (ermittelt im Frequenzintervall [0,4 THz; 1,5 THz] bzw. [0,7 THz; 1,4 THz]), ändert er sich bei prozentual gleicher Variation der anderen Rezepturanteile deutlich weniger. Weicht die Ist-Korrelation stark von der Soll-Korrelation ab, kann hierfür vor allem der Rußanteil verantwortlich sein. Auch dies ist als Fehlereingrenzungskriterium definierbar.

Darüber hinaus haben die signifikanten und charakteristischen Änderungen des Differenzenquotienten Δ(Δn/Δf) bei Ruß und Schwefel bedeutsame Vorteile. Hiermit kann eine Rezepturabweichung vom Sollwert auch dann festgestellt werden, wenn die Änderung des Brechungsindexabsolutwerts (Δn) z. B. bei einer ausgewählten Frequenz oder gemittelt in einem Frequenzfenster gleich ist, da die Konzentration zweier Bestandteile gleichzeitig gegenläufige dielelektrische Auswirkungen haben. Die Änderung der Rezepturzusammensetzung könnte bei alleiniger Betrachtung des Brechungsindexabsolutwertes nicht erkannt werden. Die Analyse der Änderung der Dispersion bietet hier eine zusätzliche Möglichkeit und damit zusätzliche Sicherheit, die Abweichungen von der Sollmischung des Materials anhand dieses Kontrollparameters aufzudecken.

In Versuchen mit Elastomeren konnte deutlich erkannt werden, dass eine starke Diversifizierung hinsichtlich der Absorption, des Brechungsindexes und der Dispersion des Brechungsindexes zwischen den Stoffsystemen Ethylen-Propylen-Dien-Kautschuk (EPDM), Naturkautschuksystemen (NR), Acrylnitril-Butadien-Kautschuk (NBR) und Chlorbutadien-Kautschuk (CR) vorliegt. Zusätzlich ist die Diversifizierung auch innerhalb der Stoffsysteme bei geringen Veränderungen der Zuschlagstoffe gegeben. Verschiedene Rezepturen lassen sich vor allem aufgrund ihres Brechungsindexes signifikant voneinander unterscheiden. Charakteristische Frequenzverläufe in Form von Fingerabdrücken, z. B. Absorptionspeaks oder eine entsprechende Dispersionsstufe in einem engen Frequenzfenster, sind dabei nicht feststellbar. Die geringste Absorption zeigen die Stoffsysteme auf NR-Basis. Auch EPDM hat bei vergleichsweise geringeren Füllstoffgehalten eine hohe THz-Transparenz. Die untersuchten NBR- und CR-Systeme absorbieren dagegen stark.

Schon geringe Änderung der Anteile der Zuschlagstoffe in den einzelnen Stoffsystemen, insbesondere der von Ruß, Schwefel, Vernetzer, Zinkoxid, Vernetzungsbeschleuniger, Fettsäuren und Stabilisatoren lassen sich in allen Fällen statistisch signifikant erfassen, da sowohl die gemessenen Mittelwerte der Korrelationen deutlich auseinander liegen, als auch die Intervalle der erweiterten Messunsicherheit (Streubereiche) nicht überlappen. Die Ergebnisse sprechen für eine hohe Genauigkeit sowie Messempfindlichkeit der dielektrischen Materialeigenschaften für elektromagnetische Wellen im THz-Bereich gegenüber einer Rezepturvariation. Sogar die absolut betrachtet geringsten Gehaltsvariationen der Fettsäuren lassen sich aufgrund ihres deutlichen dielektrischen Kontrastes zur Matrix des Stoffsystems noch nachweisen.

Zusätzlich könnte in der Auswerteeinheit wenigstens eine Fehler-Korrelation von wenigstens einer falsch zusammengesetzten Elastomermischung hinterlegt sein, und das Verfahren den folgenden Schritt umfassen:
■ Ausgabe eines Fehlersignals mit Benennung der falschen Elastomermischung, wenn die Ist-Korrelationen mit den Fehler-Korrelationen einer falschen Materialzusammensetzung übereinstimmen.

Jede Fehler-Korrelation kann wiederum aus einem einzelnen Wertepaar bestehen, das eine dielektrische Materialeigenschaft bei einer bestimmten Frequenz angibt. Vorzugsweise besteht sie jedoch aus einer Vielzahl an Wertepaaren, sodass einer Vielzahl an bestimmten Frequenzen jeweils ein Wert einer dielektrischen Materialeigenschaft zugeordnet ist. Besonders bevorzugt beschreibt die Ist-Korrelation einen lückenlosen Verlauf einer dielektrischen Materialeigenschaft über ein Frequenzspektrum. Eine solche ist beispielsweise durch Approximation, Interpolation oder andere Näherungsverfahren aus mehreren Wertepaaren erstellbar.

Stimmen die Ist-Korrelationen mit den Fehler-Korrelationen einer falschen Elastomermischung überein, ist nicht nur eine Aussage darüber treffbar, dass die Elastomermischung falsch zusammengesetzt ist, sondern auch darüber, welcher Bestandteil in welcher Menge falsch dosiert ist. Auch die Fehler-Korrelationen könnten über Bestimmungen an Referenzmischungen mit definierten falschen Dosierungen in der Auswerteeinheit hinterlegt werden. Beispielsweise können Fehler-Korrelationen aller Materialzusammensetzungen hinterlegt werden, bei denen im Verhältnis zur Soll-Zusammensetzung ein Zuschlagstoff komplett fehlt. Allerdings ist es bei einer hohen Anzahl an Bestandteilen in der Elastomermischung aufwendig alle möglichen Fehler-Korrelationen zu bestimmen und in der Auswerteeinheit zu hinterlegen. Weniger Aufwand wäre gegeben, wenn z.B. sämtliche Soll-Korrelationen von in einem Werk zu verarbeitenden Elastomermischungen in der Auswerteeinheit hinterlegt werden. Ein versehentliches Vertauschen von den Elastomermischungen wäre so aufdeckbar.

Außerdem kann mittels des Verfahrens ein Rezepturwechsel detektiert werden, indem für wenigstens zwei Elastomermischungen Soll-Korrelationen in der Auswerteeinheit hinterlegt sind.

Eine Detektion eines Rezepturwechsels ist dann gegeben, wenn die Ist-Korrelationen erst mit den Soll-Korrelationen der ersten Elastomermischung übereinstimmen und dann mit den Soll-Korrelationen der zweiten Elastomermischung übereinstimmen. Wird ein Rezepturwechsel detektiert kann das Verfahren eine Ausgabe eines den Rezepturwechsel kennzeichnenden Informationssignals vorsehen.

Somit lässt sich schnell, komfortabel und ohne Materialverschwendung ein Wechsel zwischen den zwei Elastomermischungen gewährleisten. Die Angabe eines die zweite Elastomermischung kennzeichnenden Informationssignals kann hierbei ausreichend sein. Als Zwischenschritt liegt meist eine Mischung der alten und der neuen Rezeptur vor, sodass die Ist-Korrelationen mit keiner der Soll-Korrelationen übereinstimmen. Solange dies der Fall ist, könnte als Informationssignal ein Fehlersignal ausgegeben werden.

Ergänzt werden kann das Verfahren durch einen im Bereich des Prüfraums angeordneten Temperatursensor, der mit der Auswerteeinheit verbunden ist. Hierdurch kann die Temperatur der Elastomermischung im Prüfraum bestimmt werden. Da dielektrische Materialeigenschaften abhängig von der Materialtemperatur sein können, kann so eine festgestellte Abweichung der Ist-Korrelation zur Soll-Korrelation auch auf eine fehlerhafte Temperatur der Elastomermischung zurückgeführt werden. Dem Informationssignal könnte dann zumindest als Zusatzinformation beigefügt werden, dass das Ergebnis auch auf eine falsche Temperierung zurückführbar sein könnte. Da die dielektrischen Materialeigenschaften im THz-Bereich und bei kleineren Temperaturschwankungen nur wenig Veränderung zeigen, ist die Ergänzung des Temperatursensors jedoch nicht zwingend notwendig. Zudem erfolgt die Temperaturüberwachung bei der Verarbeitung von Elastomermischungen meist schon zu anderen Qualitätssicherungszwecken. Dementsprechend kann meist von einer relativ konstanten Temperatur im Prüfraum ausgegangen werden. Ist dies jedoch nicht der Fall, stellt der Temperatursensor ein wichtiges Ausgestaltungsmittel der Erfindung dar.

Gleichsam könnte die Auswerteeinheit mit einem im Bereich des Prüfraums angeordneten Drucksensor verbunden sein. Druckbedingte Schwankungen der Abweichung zwischen der 1st- und der Soll-Korrelation können so ausgeblendet werden, z. B. durch ausschließliche Bestimmung der Ist-Korrelation bei einem bestimmten Druck. Dieses Problem könnte auch durch die Vornahme der Bestimmung zu bestimmten Zeitpunkten während eines Schließzyklus eines Werkzeugs gelöst werden. Die Auswirkung des Drucks auf die dielektrischen Materialeigenschaften im THz-Frequenzbereich ist jedoch in vielen Anwendungsfällen vernachlässigbar.

Zur Umsetzung der Erfindung kann eine Messeinrichtung vorgesehen werden, die ein THz-Spektrometer, einen von diesem definierten Prüfraum für Spektroskopiemessungen und eine Auswerteeinheit aufweist. Angeordnet werden kann solch eine Messeinrichtung in einer Fertigungsvorrichtung zum Herstellen eines Objekts aus einer plastifizierten Elastomermischung. Das Spektrometer kann die Auswerteeinheit selbst aufweisen, oder aber letztere ist separat ausgebildet. Mittels der Eintrittsseite und der Austrittsseite kann der Durchgang einfach mit einer bestehenden Leitung oder einem bestehenden Kanal strömungsverbunden werden. Somit besteht die Möglichkeit die plastifizierte Elastomermischung während der Produktion und noch vor Überführung in ein Objekt, Produkt oder Endprodukt zu prüfen.

Die Fertigungsvorrichtung zeichnet sich somit durch die unmittelbare Integration der Messeinrichtung aus, wobei die vor der Fertigung plastifizierte und somit fließfähige Elastomermischung unmittelbar im Fertigungsprozess praxisnah und genau hinsichtlich der Zusammensetzung beurteilt werden kann. Die Messeinrichtung erlaubt eine kontinuierliche Messung und es liegen keine signifikanten Messlücken zwischen einzelnen Stichproben vor. Somit kann frühzeitig festgestellt werden, ob die Qualität der Elastomermischung stimmt und die Produktion von Ausschuss ist reduzierbar. Eine personal- und zeitintensive Probenpräparation und Messung ist nicht notwendig. Außerdem kann ein schneller Rezepturwechsel mit geringen Kosten und guter Ökobilanz durchgeführt werden, indem der Zeitpunkt des Vorliegens des neuen Materials hinter der Bereitstellungseinrichtung genau bestimmt werden kann.

Durch das Verfahren erzielte Daten können zusätzlich an moderne Prozessregelsysteme übertragen werden, um so Qualitätsschwankungen bei der Fertigung zu vermeiden und Einsparungen hinsichtlich des Rohmaterial- und des Energieverbrauchs zu erzielen. All dies ist mit einem geringen Personalaufwand erreichbar. Eine effektive Qualitätskontrolle mit engen Qualitätstoleranzen und gut zertifizierbaren Bauteilen aufgrund lückenloser Überwachung wird hiermit realisierbar.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnungen. Es zeigen:
- Fig. 1: eine Fertigungsvorrichtung mit einer Messeinrichtung;
- Fig. 2: eine Messeinrichtung mit einem an einer Schlitzdüse angeordnetem THz-Spektrometer;
- Fig. 3: eine Ist-Korrelation eines Absorptionskoeffizienten über einen Frequenzbereich in Form eines Diagramms;
- Fig. 4: eine Ist-Korrelation eines Brechungsindexes über einen Frequenzbereich in Form eines Diagramms; und
- Fig. 5: eine Ist-Korrelation einer Ableitung eines Brechungsindexes über einen Frequenzbereich in Form eines Diagramms.

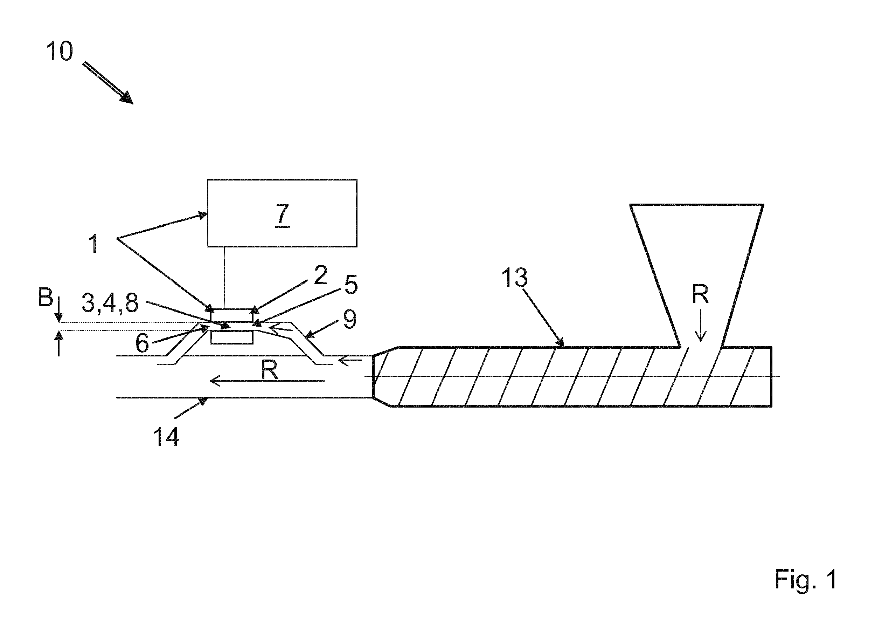

Fig. 1 zeigt schematisch eine nicht maßstäbliche Fertigungsvorrichtung 10 zum Herstellen eines Objekts aus einer plastifizierten Elastomermischung R, mit einer Messeinrichtung 1 und einem Extruder 13 zur Plastifizierung der Elastomermischung R. Insbesondere ist der Extruder 13 verkleinert dargestellt. Letzterer weist eine Schneckenwelle auf, mit der die Elastomermischung R durch Scherbeanspruchung plastifiziert und somit fließfähig wird. Ausgangsseitig schließt sich an den Extruder 13 ein Strömungskanal 14 an. Dieser ist von einer plastifizierten Elastomermischung R durchströmbar. Der Strömungskanal 14 hat einen Bypass 9, in welchem wiederum eine Schlitzdüse 8 angeordnet ist.

Die Messeinrichtung 1 verfügt über ein THz-Spektrometer 2 und eine Auswerteeinheit 7 zur Bestimmung einer Zusammensetzung der plastifizierten Elastomermischung R. Das THz-Spektrometer 2 definiert einen Prüfraum 3 für Spektroskopiemessungen. Der Prüfraum 3 hat einen Durchgang 4 mit einer Eintrittsseite 5 und einer Austrittsseite 6. Das THz-Spektrometer 2 ist derart am Bypass 9 angeordnet, dass der Durchgang 4 von der Eintrittsseite 5 zur Austrittsseite 6 von der plastifizierten und somit fließfähigen Elastomermischung R durchströmbar ist. Somit ist der Extruder 13 mit der Materialeintrittsseite 5 des Durchgangs 4 strömungsverbunden. Insbesondere ist der Durchgang 4 Teil der in den Bypass 9 integrierten Schlitzdüse 8. Hierdurch ergibt sich eine relativ geringe Querschnittsbreite B im Bereich des Durchgangs 4. Diese sollte kleiner als 2,5 mm sein und besonders bevorzugt 1 +- 0,2 mm betragen. Außerdem ist sie wie dargestellt in Strömungsrichtung der Elastomermischung R über die gesamte Länge des Durchgangs 4 konstant.

Das Spektrometer 2 weist einen Emitter und einen gegenüberliegend auf der anderen Seite der Schlitzdüse 3 angeordneten Empfänger auf und ist mit einer Auswerteeinheit 7 verbunden. In letzterer ist wenigstens eine Soll-Korrelation von wenigstens einer Elastomermischung R hinterlegt, wobei die Soll-Korrelationen jeweils eine dielektrische Materialeigenschaft der Elastomermischung R in Abhängigkeit einer Frequenz von sich in der Elastomermischung R ausbreitenden elektromagnetischen Wellen beschreiben.

Unter Zuhilfenahme einer derartigen Messeinrichtung 1 kann ein Verfahren zur Bestimmung einer Zusammensetzung einer plastifizierten Elastomermischung R auf der Basis wenigstens eines polaren oder unpolaren Kautschuks durchgeführt werden, welches folgende Schritte umfasst:
- Bestrahlen der plastifizierten Elastomermischung R auf der Basis des wenigstens einen polaren oder unpolaren Kautschuks in dem Prüfraum 3 des THz-Spektrometers 2 mit elektromagnetischen Wellen im THz-Frequenzbereich;
- Bestimmen von wenigstens einer Ist-Korrelation zwischen der Frequenz der emittierten elektromagnetischen Wellen und einer dielektrischen Materialeigenschaft der Elastomermischung R anhand von empfangenen elektromagnetischen Wellen;
- Vergleichen der Ist-Korrelationen mit Soll-Korrelationen mittels der Auswerteeinheit 7, wobei in der Auswerteeinheit 7 wenigstens eine Soll-Korrelation von wenigstens einer Elastomermischung R hinterlegt ist;
- Ausgabe eines Informationssignals, wenn wenigstens eine Ist-Korrelation von der Soll-Korrelation der Elastomermischung R abweicht.

Hierfür kann die Messeinrichtung 1 durch folgende Schritte kalibriert werden:
a) Hinterlegen einer ermittelten Ist-Korrelation als Soll-Korrelation einer Referenzmischung in der Auswerteeinheit 7.

Weiterhin könnte in der Auswerteeinheit 7 wenigstens eine Fehler-Korrelation von wenigstens einer falschen Elastomermischung hinterlegt sein, und das Verfahren folgenden Schritt umfassen:
■ Ausgabe eines Fehlersignals mit Benennung der falschen Elastomermischung, wenn die Ist-Korrelationen mit den Fehler-Korrelationen einer falschen Elastomermischung übereinstimmen.

Gemäß einer Weiterbildung könnten in der Auswerteeinheit 7 Fehler-Eingrenzungskriterien hinterlegt sein, und nach dem Vergleichen der Ist-Korrelationen mit den Soll-Korrelationen können zwischen diesen ermittelte Abweichungen anhand dieser definierten Fehlereingrenzungskriterien interpretiert werden.

Letztlich kann mit Hilfe einer Ausbildung des Verfahrens ein verbesserter Rezepturwechsel durchgeführt werden, indem für wenigstens zwei Elastomermischungen R Soll-Korrelationen in der Auswerteeinheit 7 hinterlegt sind. Der Rezepturwechsel ist dann detektierbar, wenn die Ist-Korrelationen erst mit den Soll-Korrelationen der ersten Elastomermischung R übereinstimmen und dann mit den Soll-Korrelationen der zweiten Elastomermischung übereinstimmen. Nach Detektion sollte eine Ausgabe eines den Rezepturwechsel kennzeichnenden Informationssignals erfolgen.

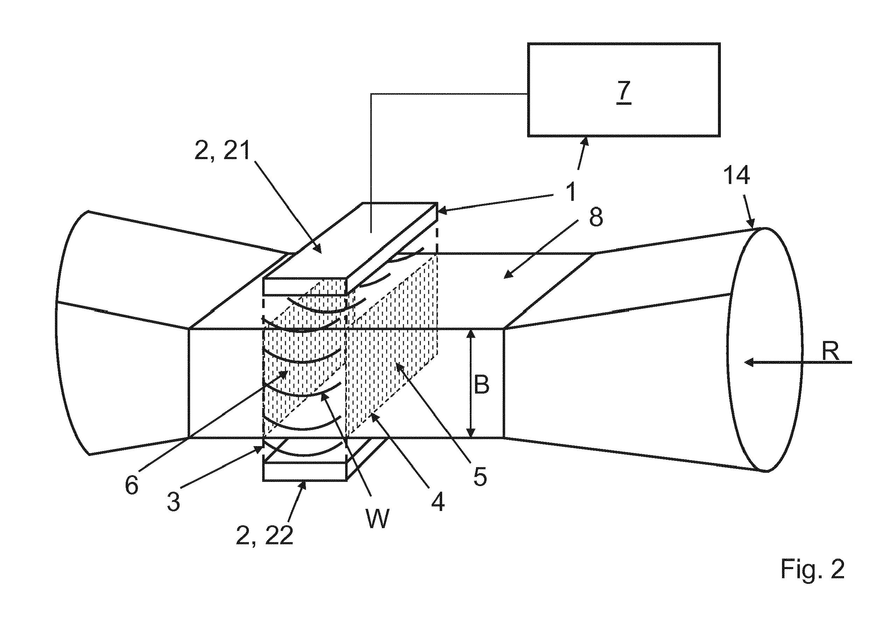

Fig. 2 zeigt eine nicht maßstabsgetreue Schemaskizze einer Messeinrichtung 1 mit einem an einer Schlitzdüse 8 angeordneten THz-Spektrometer 2. Die Schlitzdüse 8 verjüngt einen Strömungskanal 14 auf eine Querschnittsbreite B, um die Materialstärke in einem Prüfraum 3 des THz-Spektrometers 2 zu verringern. Insbesondere bildet die Schlitzdüse 8 einen Durchgang 5 durch den Prüfraum 3. Wie erkennbar, ist der Prüfraum 3 dabei größer als der Durchgang 5, da ein Emitter 21 und ein Empfänger 22 des THz-Spektrometers 2 jeweils beabstandet zur Schlitzdüse 8 und sich gegenüberliegend angeordnet sind. Dieser Abstand kann zur Unterdrückung von Absorption durch Wasser mit einem trockenen Gas, z.B. getrockneter Luft oder Stickstoff gefüllt oder mit einem Vakuum beaufschlagt werden. Mithin liegt der Durchgang 4 innerhalb des Prüfraums 3, nicht jedoch der Prüfraum 3 innerhalb des Durchgangs 4. Außerdem ist die Schlitzdüse 8 zwischen dem Emitter 21 und dem Empfänger 22 angeordnet. In dem Zwischenraum zwischen dem Emitter 21, bzw. dem Empfänger 22 und dem Durchgang 4 kann zum Beispiel ein Trennelement, insbesondere aus einem schwach absorbierenden Material angeordnet werden. Der Zwischenraum kann jedoch auch eine thermische Trennung des Strömungskanals 14 und des Emitters 21 sowie des Empfängers 22 bewirken. In Messversuchen erwies sich ein Abstand des Empfängers 22 von 30 cm vom Durchgang 5 als besonders geeignet, wobei sowohl der Emitter 21 als auch der Empfänger 22 eine zum Durchgang 5 ausgerichtete Grundfläche von circa 10 mm x10 mm hatten. Die Querschnittsbreite B betrug in den Messversuchen 1 mm.

Durch die verringerte Querschnittsbreite B können elektromagnetische Wellen W im THz-Frequenzbereich auch stark absorbierende Elastomermischungen R durchdringen und erlauben eine Bestimmung von deren Zusammensetzung. Außerdem ist die Querschnittsbreite B im gesamten Bereich des Durchgangs 4 konstant. Dementsprechend ist auch die Materialstärke einer im Prüfraum 3 befindlichen Elastomermischung R konstant haltbar. Dies ist wichtig für schnell und genau durchzuführende Spektroskopiemessungen. Zur Auswertung von mit dem Empfänger 22 empfangenen elektromagnetischen Wellen W, sind der Emitter 21 und der Empfänger 22 mit einer Auswerteeinheit 7 verbunden. In dieser können Soll-Korrelationen hinterlegt sein.

Eine Elastomermischung R ist nunmehr durch den Strömungskanal 14 leitbar und wird in diesem durch die Schlitzdüse 8 auf eine Querschnittsbreite B verjüngt. Im Bereich der Schlitzdüse 8 tritt die Elastomermischung R durch eine Eintrittsseite 5 des Durchgangs 4 in den Prüfraum 3 ein, durchquert diesen und verlässt den Prüfraum 3 schließlich durch eine Austrittsseite 6 des Durchgangs 4. Während der Durchleitung durch den Prüfraum 3 ist nunmehr eine Ist-Korrelation dielektrischer Materialeigenschaften der Elastomermischung R mittels dem THz-Spektrometer 2 und der Auswerteeinheit 7 bestimmbar. Schließlich weitet sich die Schlitzdüse 8 hinter der Austrittsseite 6 des Durchgangs 4 wieder auf den Querschnitt des ursprünglichen Strömungskanals 14 auf.

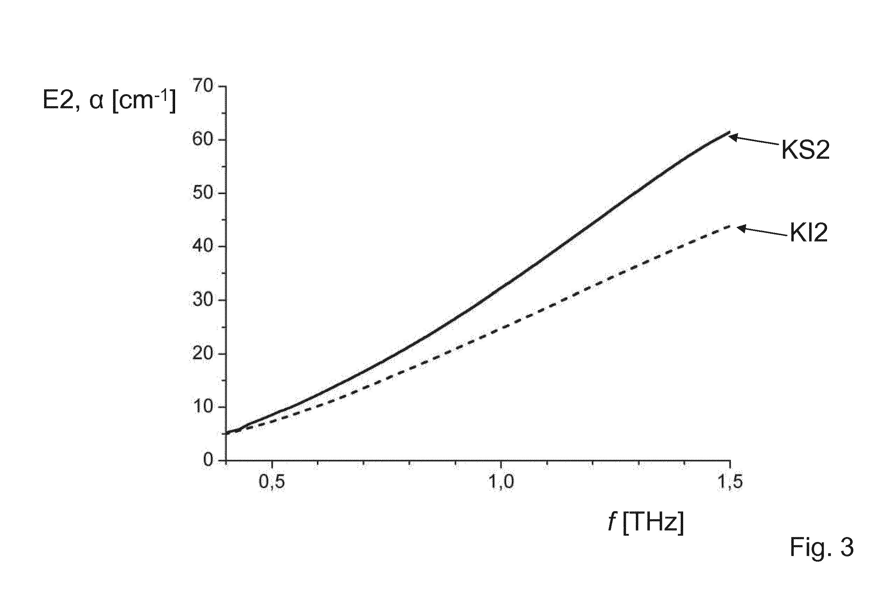

Fig. 3 zeigt ein Diagramm, auf dessen Abszisse eine Frequenz f in der Einheit THz und auf dessen Ordinate eine Absorption E2, insbesondere ein Absorptionskoeffizient α in der Einheit cm⁻¹, aufgetragen sind. In dem Diagramm finden sich nunmehr zwei Graphen. Der erste Graph beschreibt eine zweite Soll-Korrelation KS2 und der erste Graph eine zweite Ist-Korrelation KI2. Dabei ist klar erkennbar, dass die Absolutwerte der zweiten Ist-Korrelation KI2 über den gezeigten Frequenzbereich kleiner sind als die Werte der zweiten Soll-Korrelation KS2. Zudem ist die Dispersion, d.h. die Steigung, der zweiten Ist-Korrelation KI2 geringer als die der zweiten Soll-Korrelation KS2. Dies bedeutet, dass die Absorption einer durch die zweite Ist-Korrelation KI2 beschriebenen Elastomermischung R weniger stark von der Frequenz elektromagnetischer Wellen abhängt als sie es gemäß der zweiten Soll-Korrelation sollte. Ein solches Verhältnis zwischen den zweiten Korrelationen KI2, KS2 liegt beispielsweise bei einer relativ um 10% zu geringen Beimischung des Zuschlagsstoffs Ruß in einer Elastomermischung auf Naturkautschukbasis vor.

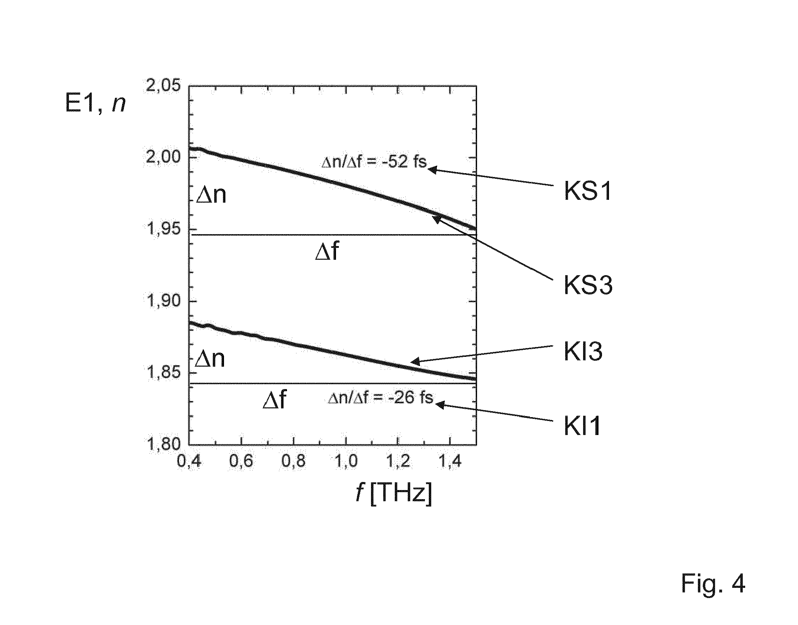

Fig. 4 zeigt ein weiteres Diagramm. Auf dessen Abszisse ist wiederum eine Frequenz f mit der Einheit THz abgebildet und auf der Ordinate ist eine Ausbreitungsgeschwindigkeit E1 in der physikalischen Größe des Brechungsindexes n aufgetragen. Der Brechungsindex n gibt an, um welchen Faktor sich eine elektromagnetische Welle innerhalb eines Materials langsamer ausbreitet als im Vakuum. In dem Diagramm sind zwei Graphen eingezeichnet, wobei einer von diesen eine dritte Soll-Korrelation KS3 und der andere eine dritte Ist-Korrelation KI3 beschreiben. Dabei liegen die absoluten Werte der dritten Ist-Korrelation KI1 über den gesamten betrachteten Frequenzbereich f unterhalb der Werte der dritten Soll-Korrelation KS3. Die Werte des Brechungsindexes n beider dritten Korrelationen KI3, KS3 sinken mit zunehmender Frequenz f. Das bedeutet, dass je höher die Frequenz f einer elektromagnetischen Welle ist, desto weniger stark wird sie von der Elastomermischung abgebremst, d.h. desto höher ist ihre Ausbreitungsgeschwindigkeit in der Elastomermischung.

Die dritten Korrelationen verlaufen hierbei jedoch nicht parallel zueinander. Somit unterscheidet sich ihre Dispersion. Die Dispersion ist als erste Ist-Korrelation KI1 und als erste Soll-Korrelation KS1 gezeigt. Insbesondere sind die ersten Korrelationen KI1, KS1 durch einen Differenzenquotienten gebildet, der sich aus der Änderung des Brechungsindexes Δn über einen Frequenzbereich Δf ergibt. Wie hier dargestellt umfasst dieser Frequenzbereich 4f den gesamten auf der Abszisse dargstellten Frequenzbereich. Hierdurch werden Schwankungen der Steigung über den Frequenzbereich f nicht berücksichtigt. Dies ist besonders vorteilhaft, da die Steigung des Graphen über kleine Ausschnitte des Frequenzbereichs mitunter sehr stark schwankt. Insbesondere schwankt die Steigung deutlich mehr als die Absolutwerte des Brechungsindexes n. Die Ausbildung des Differenzenquotienten Δn/Δf macht einen fehlerfreien Vergleich der Dispersion zwischen den ersten Korrelationen KI1, KS1 möglich.

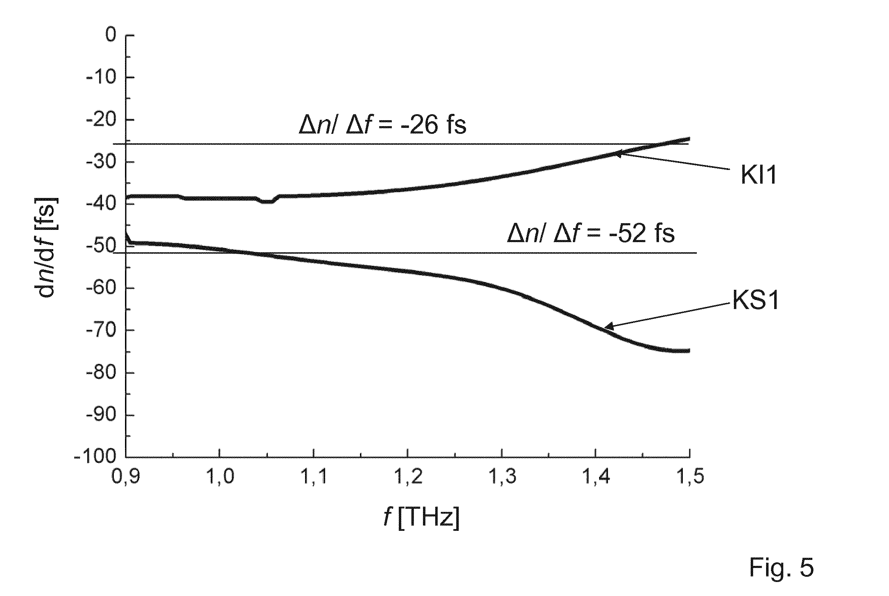

Fig. 5 zeigt eine erste Ist-Korrelation KI1, ausgebildet durch eine Ableitung eines Brechungsindexes über einen Frequenzbereich dn/df in Form eines Diagramms. Auf der Abszisse des Diagramms ist eine Frequenz f mit der Einheit THz abgebildet und auf der Ordinate ist die Ableitung des Brechungsindexes über die Frequenz d*n*/d*f* mit der Einheit fs aufgetragen. Wie man erkennt, liegt die erste Ist-Korrelation KI1 über den gesamten betrachteten Frequenzbereich oberhalb einer ersten Soll-Korrelation KS1. Dies bedeutet, dass die Dispersion des Brechungsindex einer mit der ersten Ist-Korrelation KI1 beschriebenen Elastomermischung über den gesamten betrachteten THz-Frequenzbereich weniger stark von der Frequenz der elektromagnetischen Wellen abhängig ist als eine mit der ersten Soll-Korrelation KS1 beschriebene Elastomermischung.

Weiterhin sind im Diagramm Differenzenquotienten Δn/Δf des Brechungsindex n über den gesamten Frequenzbereich gezeigt. Dieser ist gewissermaßen der Mittelwert der Ableitung des Brechungsindexes über die Frequenz d*n*/d*f*. Bereits der Unterschied zwischen dem Differenzenquotient Δn/Δf der ersten Ist-Korrelation KI1 und dem Differenzenquotient Δn/Δf der ersten Soll-Korrelation KS1 ermöglicht eine Detektion einer Abweichung von einer Elastomermischung. Gegebenenfalls kann auch über die Höhe des Unterschieds der Fehler in der Elastomermischung eingegrenzt werden. Zum Beispiel können Rezepturbestandteile ausgeschlossen werden, die eine solch hohe Abweichung überhaupt nicht alleine verursachen können.

Mit einer Betrachtung der Ableitung des Brechungsindexes über die Frequenz d*n*/d*f* gelingt eine deutlich schärfere Eingrenzung, da auch der Verlauf über die Frequenz f zur Beurteilung der falschen Elastomermischung heranziehbar ist. Im gezeigten Beispiel ändert sich der Brechungsindex der tatsächlich vorliegenden Elastomermischung mit zunehmender Frequenz f nur noch sehr wenig (vgl. KI1), wohingegen sich dieser eigentlich mit zunehmender Frequenz f sehr viel stärker bei höheren Frequenzen f ändern sollte. Eine ähnlich gute Eingrenzung lässt sich erzielen, wenn mehrere Differenzenquotienten Δn/Δf des Brechungsindex n über Teil-Frequenzbereiche gebildet werden, z. B. über 0,1 THz-Frequenzbereiche.

Die Erfindung ist nicht auf eine der vorbeschriebenen Ausführungsformen beschränkt, sondern in vielfältiger Weise abwandelbar.

Sämtliche aus den Ansprüchen, der Beschreibung und der Zeichnung hervorgehenden Merkmale und Vorteile, einschließlich konstruktiver Einzelheiten, räumlicher Anordnungen und Verfahrensschritten, können sowohl für sich als auch in den verschiedensten Kombinationen erfindungswesentlich sein.

### Bezugszeichenliste

- 1: Messeinrichtung
- 2: THz-Spektrometer
- 21: Emitter
- 22: Empfänger
- 3: Prüfraum
- 4: Durchgang
- 5: Eintrittsseite
- 6: Austrittsseite
- 7: Auswerteeinheit
- 8: Schlitzdüse
- 9: Bypass
- 10: Fertigungsvorrichtung
- 13: Extruder
- 14: Strömungskanal

- α: Absorptionskoeffizient

- B: Querschnittsbreite

- E1: Ausbreitungsgeschwindigkeit
- E2: Absorption
- f: Frequenz
- n: Brechungsindex

- KI1: erste Ist-Korrelation
- KI1: zweite Ist-Korrelation
- KI1: dritte Ist-Korrelation
- KS1: erste Soll-Korrelation
- KS2: zweite Soll-Korrelation
- KS3: dritte Soll-Korrelation

- R: Elastomermischung
- W: elektromagnetische Wellen

## Patentansprüche

1. Verfahren zur Bestimmung einer Zusammensetzung einer plastifizierten Elastomermischung (R) auf der Basis wenigstens eines polaren oder unpolaren Kautschuks, umfassend die folgenden Schritte:
b) Bestrahlen der plastifizierten Elastomermischung (R) auf der Basis des wenigstens einen polaren oder unpolaren Kautschuks in einem Prüfraum (3) eines THz-Spektrometers (2) mit elektromagnetischen Wellen (W) im THz-Frequenzbereich;
c) Bestimmen von wenigstens einer Ist-Korrelation (KI1, KI2, KI3) zwischen der Frequenz (f) der emittierten elektromagnetischen Wellen (W) und einer dielektrischen Materialeigenschaft der Elastomermischung (R) anhand von empfangenen elektromagnetischen Wellen (W);
d) Vergleichen der Ist-Korrelationen (KI1, KI2, KI3) mit Soll-Korrelationen (KS1, KS2, KS3) mittels einer Auswerteeinheit (7), wobei in der Auswerteeinheit (7) wenigstens eine Soll-Korrelation (KS1, KS2, KS3) von wenigstens einer Elastomermischung (R) hinterlegt ist;
e) Ausgabe eines Informationssignals, wenn wenigstens eine Ist-Korrelation (KI1, KI2, KI3) von der Soll-Korrelation (KS1, KS2, KS3) der Elastomermischung (R) abweicht.

2. Verfahren nach Anspruch 1, **dadurch gekennzeichnet, dass** eine erste Ist-Korrelation (KI1) und eine zugehörige erste Soll-Korrelation (KS1) jeweils eine spektrale Dispersion einer dielektrischen Materialeigenschaft beschreiben.

3. Verfahren nach Anspruch 2, **dadurch gekennzeichnet, dass** die spektrale Dispersion eine frequenzabhängige Änderung einer Ausbreitungsgeschwindigkeit (E1) über der Frequenz (f), von sich in der Elastomermischung (R) ausbreitenden elektromagnetischen Wellen (W) umfasst.

4. Verfahren nach einem der Ansprüche 2 oder 3, **dadurch gekennzeichnet, dass** die spektrale Dispersion einen Differenzenquotienten aus einem Brechungsindex (n) der Elastomermischung (R) und der Frequenz (f) von sich in der Elastomermischung (R) ausbreitenden elektromagnetischen Wellen (W) umfasst.

5. Verfahren nach einem der vorhergehenden Ansprüche, **dadurch gekennzeichnet, dass** eine zweite Ist-Korrelation (KI2) und eine zugehörige zweite Soll-Korrelation (KS2) jeweils eine Absorption (E2) elektromagnetischer Wellen (W) durch die Elastomermischung (R) in Abhängigkeit von der Frequenz (f) beschreiben.

6. Verfahren nach einem der vorhergehenden Ansprüche, **dadurch gekennzeichnet, dass** eine dritte Ist-Korrelation (KS3) und eine zugehörige dritte Soll-Korrelation (KS3) eine Ausbreitungsgeschwindigkeit (E1) von elektromagnetischen Wellen (W) in der Elastomermischung (R) in Abhängigkeit von der Frequenz (f) beschreiben.

7. Verfahren nach einem der vorhergehenden Ansprüche, **dadurch gekennzeichnet, dass** die plastifizierte Elastomermischung (R) während der Bestimmung der Ist-Korrelationen (KI1, KI2, KI3) durch einen Durchgang (4) des Prüfraums (3) hindurch geleitet wird.

8. Verfahren nach Anspruch 7, **dadurch gekennzeichnet, dass** ein Extruder (13) mit dem Durchgang (4) des Prüfraums (3) strömungsverbunden ist.

9. Verfahren nach einem der vorhergehenden Ansprüche, **gekennzeichnet durch** folgende Schritte:
f) Hinterlegen einer ermittelten Ist-Korrelation (KI1, KI2, KI3) als Soll-Korrelation (KS1, KS2, KS3) einer Referenzmischung in der Auswerteeinheit (7).

10. Verfahren nach einem der vorhergehenden Ansprüche, **dadurch gekennzeichnet, dass** nach dem Vergleichen der Ist-Korrelationen (KI1, KI2, KI3) mit den Soll-Korrelationen (KS1, KS2, KS3) zwischen diesen ermittelte Abweichungen anhand von definierten Fehlereingrenzungskriterien interpretiert werden.

11. Verfahren nach einem der vorhergehenden Ansprüche, **dadurch gekennzeichnet, dass** für wenigstens zwei Elastomermischung (R) Soll-Korrelationen (KS1, KS2, KS3) in der Auswerteeinheit (7) hinterlegt sind.
